(19) Europäisches Patentamt
European Patent Office
Office européen des brevets

(11) Veröffentlichungsnummer: **0 014 455**
**A2**

(12) **EUROPÄISCHE PATENTANMELDUNG**

(21) Anmeldenummer: 80100548.9

(22) Anmeldetag: 04.02.80

(51) Int. Cl.[3]: **C 07 C 69/74**
C 07 C 67/14, C 07 C 67/10
C 07 C 121/75, C 07 C 153/09
C 07 C 79/24, A 01 N 53/00
//C07C79/32, C07C43/215,
C07C33/28

(30) Priorität: 09.02.79 CH 1290/79
18.05.79 CH 4674/79
11.01.80 CH 214/80

(43) Veröffentlichungstag der Anmeldung:
20.08.80 Patentblatt 80/17

(84) Benannte Vertragsstaaten:
BE CH DE FR GB IT LU NL SE

(71) Anmelder: F.HOFFMANN-LA ROCHE & CO.
Aktiengesellschaft

CH-4002 Basel(CH)

(72) Erfinder: Suchy, Milos, Dr.
Grossplatzstrasse 3
CH-8122 Pfaffhausen(CH)

(74) Vertreter: Lederer, Franz, Dr. et al,
Patentanwälte Dr. Franz Lederer Reiner F. Meyer
Lucile-Grahn-Strasse 22
D-8000 München 80(DE)

(54) **Cyclopropan(thiol)carbonsäureester, Verfahren zu deren Herstellung, Schädlingsbekämpfungsmittel enthaltend solche Verbindungen, sowie Verwendung solcher Verbindungen als Schädlingsbekämpfungsmittel.**

(57) Die Erfindung betrifft Cyclopropan(thiol)carbonsäureester der allgemeinen Formel

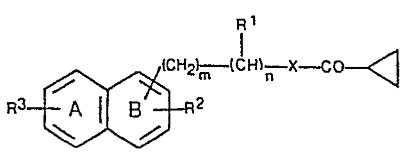

worin $R^1$ nieder Alkyl, mit Halogen substituiertes nieder Alkyl, nieder Alkenyl, nieder Alkinyl, Cyan oder Phenyl, wobei der Phenylrest gegebenenfalls mit Halogen, Methyl, Aethyl, Methoxy, Aethoxy und/oder Nitro substituiert sein kann; $R^2$ Wasserstoff, Halogen, Alkyl mit 1 bis 3 Kohlenstoffatomen, Alkoxy mit 1 bis 3 Kohlenstoffatomen, Alkenyloxy mit 3 oder 4 Kohlenstoffatomen, Alkinyloxy mit 3 oder 4 Kohlenstoffatomen oder Nitro; $R^3$ Wasserstoff, Alkyl mit 1 bis 3 Kohlenstoffatomen oder Alkoxy mit 1 bis 3 Kohlenstoffatomen; X Sauerstoff oder Schwefel; m die Zahl 0, 1, 2 oder 3; und n die Zahl 0 oder 1; bedeuten und einer der beiden Ringe A und B oder beide auch gesättigt sein können; wobei jedoch die beiden Symbole m und n aber nicht gleichzeitig die Zahl 0 darstellen; mit der Massgabe, dass die beiden Symbole $R^2$ und $R^3$ nicht gleichzeitig Wasserstoff sind, wenn X Sauerstoff und m die Zahl 1 darstellen und die den Cyclopropan(thiol)carbonsäurerest tragende Methylengruppe mit der ß-Stellung des Naphthalinrestes verknüpft ist, Verfahren zu deren Herstellung, Schädlingsbekämpfungsmittel, die diese Verbindungen als Wirkstoffe enthalten, sowie die Verwendung solcher Verbindungen und Mittel zur Bekämpfung von Schädlingen.

EP 0 014 455 A2

4. Feb. 1980 14455

F.Hoffmann-La Roche & Co.Aktiengesellschaft,Basel, Schweiz

RAN 6101/94k

## Cyclopropan(thiol)carbonsäureester, Verfahren zu deren Herstellung, Schädlingsbekämpfungsmittel enthaltend solche Verbindungen, sowie Verwendung solcher Verbindungen als Schädlingsbekämpfungsmittel

Die Erfindung betrifft Cyclopropan(thiol)carbonsäureester, nämlich Cyclopropan(thiol)carbonsäure-naphthyl-(bzw. tetrahydro- oder decahydronaphthyl)alkylester der Formel

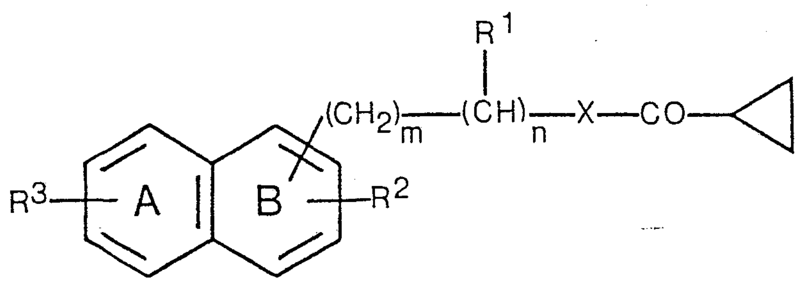

I

worin $R^1$ nieder Alkyl, mit Halogen substituiertes nieder Alkyl, nieder Alkenyl, nieder Alkinyl, Cyan oder Phenyl, wobei der Phenylrest gegebenenfalls mit Halogen, Methyl, Aethyl, Methoxy, Aethoxy und/oder Nitro substituiert sein kann; $R^2$ Wasserstoff, Halogen, Alkyl mit 1 bis 3 Kohlenstoffatomen, Alkoxy mit 1 bis 3 Kohlenstoffatomen, Alkenyloxy mit 3 oder 4 Kohlenstoffatomen, Alkinyloxy mit 3 oder 4 Kohlenstoffatomen oder Nitro; $R^3$ Wasserstoff, Alkyl mit 1 bis 3 Kohlenstoffatomen oder Alkoxy mit 1 bis 3 Kohlenstoffatomen; X Sauerstoff oder Schwefel; m die Zahl 0, 1, 2 oder 3; und n die Zahl 0 oder 1; bedeuten und einer der beiden Ringe A und B oder beide

Pa/21.11.1979

auch gesättigt sein können; wobei jedoch die beiden Symbole m und n nicht gleichzeitig die ganze Zahl 0 darstellen; mit der Massgabe, dass die beiden Symbole $R^2$ und $R^3$ nicht gleichzeitig Wasserstoff sind, wenn X Sauerstoff und m die Zahl 1 darstellen und die den Cyclopropan(thiol)carbonsäurerest tragende Methylengruppe mit der ß-Stellung des Naphthalinrestes verknüpft ist.

Die Verbindungen der Formel I sind Schädlingsbekämpfungsmittel und eignen sich insbesondere zur Bekämpfung von Insekten und Acarinen. Somit umfasst die Erfindung auch Schädlingsbekämpfungsmittel, welche die Verbindungen der Formel I enthalten, Verfahren zur Herstellung derartiger Mittel sowie deren Verwendung.

Der Ausdruck "nieder Alkyl" umfasst Alkylreste mit 1 bis 6 Kohlenstoffatomen wie Methyl, Aethyl, Propyl, Isopropyl, Butyl, Isobutyl und tert.-Butyl.

Der Ausdruck "nieder Alkenyl" umfasst geradkettige und verzweigte Alkenylreste mit 2 bis 6 Kohlenstoffatomen wie Vinyl, Propenyl, Butenyl, Isobutenyl, Pentenyl und dgl. Der Ausdruck "nieder Alkinyl" umfasst geradkettige oder verzweigte Alkinylreste mit bis zu 6 Kohlenstoffatomen wie Propargyl, Butinyl, Isobutinyl, Pentinyl und dgl.

Der Ausdruck "Halogen" umfasst, wenn nicht anderweitig angegeben, Fluor, Chlor, Brom und Jod.

Eine interessante Untergruppe von Verbindungen der Formel I besteht aus den Verbindungen der obigen allgemeinen Formel I mit der Massgabe, dass die beiden Symbole $R^2$ und $R^3$ nicht gleichzeitig Wasserstoff sind, wenn m die Zahl 1 darstellt und die den Cyclopropan(thiol)-carbonsäurerest tragende Methylengruppe mit der ß-Stellung des Naphthalinrestes verknüpft ist.

Bevorzugte Verbindungen der Formel I sind solche, worin X in der Bedeutung Sauerstoff vorliegt. Besonders bevorzugte Verbindungen der Formel I sind solche, worin $R^2$ in der Bedeutung Wasserstoff oder Halogen vorliegt. Ebenfalls bevorzugt sind Verbindungen der Formel I, worin $R^3$ Wasserstoff oder eine Methylgruppe in der 6-Stellung des Naphthalinrestes bedeutet.

Ganz besonders bevorzugte Verbindungen sind:

Cyclopropancarbonsäure-α-2-naphthylbenzylester,
Cyclopropancarbonsäure-(6-methyl-2-naphthyl)methylester,
Cyclopropanthiolcarbonsäure-2-naphthylmethylester.

Die Erfindung betrifft ferner ein Verfahren zur Herstellung der Verbindungen der Formel I. Das erfindungsgemässe Verfahren ist dadurch gekennzeichnet, dass man

a) die Säure oder ein reaktionsfähiges Derivat dieser Säure der Formel

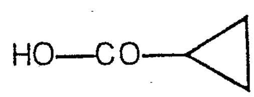

II

mit einem Alkohol bzw. Thioalkohol der Formel

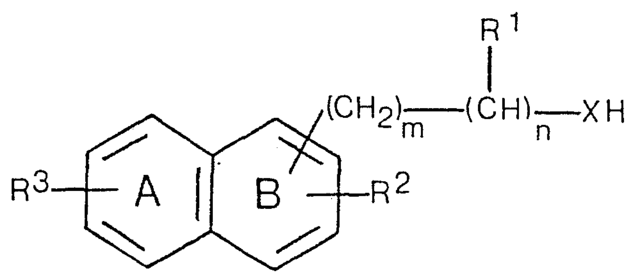

III

worin $R^1$, $R^2$, $R^3$, X, A, B, m und n die oben angegebenen Bedeutungen besitzen,

notwendigenfalls in Gegenwart einer Base umsetzt, oder

b) die Säure der Formel II oder die entsprechende Thiolsäure mit einer Verbindung der Formel

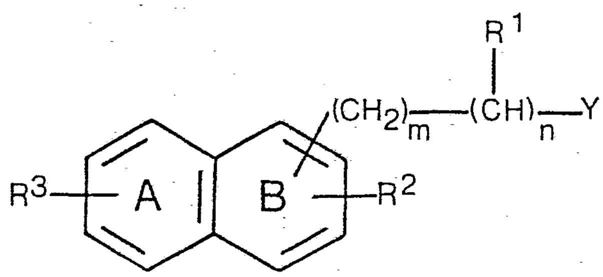

IV

worin $R^1$, $R^2$, $R^3$, A, B, m und n die oben angegebenen Bedeutungen besitzen und Y Chlor, Brom, Mesyloxy oder Tosyloxy bedeutet,

zweckmässigerweise in Gegenwart einer Base umsetzt.

In einer Ausführungsform zur Herstellung der Verbindungen der Formel I wird eine Säure der Formel II oder ein reaktionsfähiges Derivat dieser Säure mit einem Alkohol bzw. Thioalkohol der Formel III umgesetzt.

Der Ausdruck "reaktionsfähiges Derivat der Säure" bedeutet ein Säurehalogenid, ein Säureanhydrid, ein Imidazolid, einen Ester eines niedrig siedenden Alkohols, ein Alkalimetallsalz, ein Silbersalz oder ein Salz eines tertiären Amins.

Zur Herstellung der Verbindungen der Formel I wird die Veresterung der Säure der Formel II vorzugsweise in einem geeigneten inerten Lösungsmittel bei Raumtemperatur oder erhöhter Temperatur und unter geeigneten wasserabspaltenden Bedingungen, z.B. in Gegenwart von Dicyclohexylcarbodiimid oder durch azeotropes Abdestillieren des entstehenden Wassers aus dem katalysierten Reaktionsgemisch durchgeführt. Bei Verwendung eines Säurehalogenids als reaktionsfähigen Derivats der Säure wird die Umsetzung mit dem Alkohol, vorzugsweise bei Raumtemperatur und in Gegenwart eines Säureacceptors, z.B. eines tertiären Amins, wie Pyridin oder Triäthylamin, durchgeführt. Man erhält in hoher Ausbeute die entsprechenden Ester. Als Säurehalogenide werden die Säurechloride bevorzugt. Die Umsetzung wird vorzugsweise in Gegenwart eines inerten Lösungsmittels durchgeführt, wie Benzol, Toluol oder Petroläther.

Bei Verwendung eines Esters aus der Säure der Formel II und einem niedrig siedenden Alkohol, wie Methanol oder Aethanol, als reaktionsfähigen Derivats der Säure kann der entsprechende Ester der Formel I durch Erhitzen des Esters mit dem Alkohol bzw. Thioalkohol der Formel III in Gegenwart einer Base, vorzugsweise des Alkalimetallalkoholats, das dem niedrig siedenden Alkohol des eingesetzten Esters entspricht, oder in Gegenwart von Natriumhydrid in einem inerten Lösungsmittel, wie Toluol, und unter Abtrennung des bei der Umsetzung freigesetzten niedrig siedenden Alkohols durch fraktionierte Destillation, hergestellt werden.

Die Ester der Formel I können durch Umsetzen des Imidazolides einer Säure der Formel II mit dem Alkalimetallalkoholat des Alkohols bzw. Thioalkohols der Formel III, gegebenenfalls mit einer katalytischen Menge eines Alkalimetallalkoholats, z.B. Natriumäthanolat, in guter Ausbeute hergestellt werden. Die Reaktion wird vorzugsweise in einem inerten Lösungsmittel, wie Tetrahydrofuran oder Dimethoxyäthan, bei Raumtemperatur durchgeführt.

Bei Verwendung eines Säureanhydrids als reaktionsfähigen Derivats der Säure der Formel II kann der Ester der Formel I durch Umsetzen des Säureanhydrids mit dem Alkohol bzw. Thioalkohol der Formel III bei Raumtemperatur oder vorzugsweise unter Erhitzen und in Gegenwart eines Lösungsmittels, wie Toluol oder Xylol, hergestellt werden.

In einer weiteren Ausführungsform des erfindungsgemässen Verfahrens wird die Säure der Formel II bzw. die entsprechende Thiolsäure mit einem Halogenid oder Sulfonsäureester der Formel IV umgesetzt. Die Umsetzung erfolgt vorzugsweise in einem organischen Lösungsmittel. Für die Herstellung der Verbindungen der Formel I, worin X Sauerstoff bedeutet, sind beispielsweise Aceton, Methyläthylketon, Diäthylketon, Dimethylformamid, Dimethylsulfoxid, Benzol und Toluol geeignete Lösungsmittel. Für die Herstellung der Verbindungen der Formel I, worin X Schwefel bedeutet, sind beispielsweise Aceton, Methyläthylketon, Diäthylketon und Alkohol geeignete Lösungsmittel. In beiden Fällen erfolgt die erfindungsgemässe Umsetzung zweckmässigerweise in Gegenwart einer Base, vorzugsweise Kaliumcarbonat oder, insbesondere zur Herstellung der Verbindungen der Formel I, worin X Schwefel bedeutet, eines Alkalihydroxids wie Natriumhydroxid. Diese Reaktion erfolgt vorzugsweise in einem Temperaturbereich zwischen 20°C und dem Siedepunkt des Lösungsmittels. Danach wird beispielsweise das Reaktionsgemisch zum Aufarbeiten in eine anorganische Säure gegossen, vorzugsweise in verdünnte Salzsäure, verdünnte Schwefelsäure, verdünnte Phosphorsäure oder verdünnte Salpetersäure. Die saure Lösung wird mit Hexan oder Aether extrahiert und das Lösungsmittel abdestilliert. Der Rückstand wird an Kieselgel oder Aluminiumoxid chromatographiert und/oder destilliert.

Die Ausgangsmaterialien der Formel

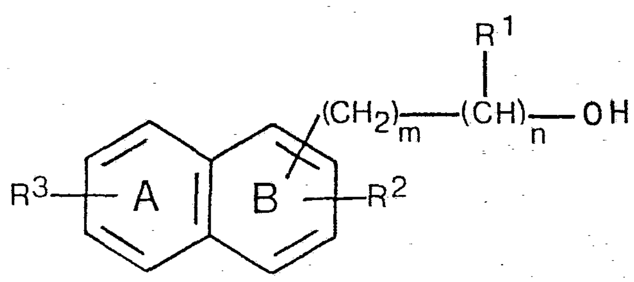

III'

worin $R^1$, $R^2$, $R^3$, A, B, m und n die oben angegebenen Bedeutungen besitzen,

sind zum Teil neu.

Für den Fall, dass das Symbol n die Zahl 1 bedeutet, können diese Verbindungen der Formel III' beispielsweise hergestellt werden, indem man eine Verbindung der Formel

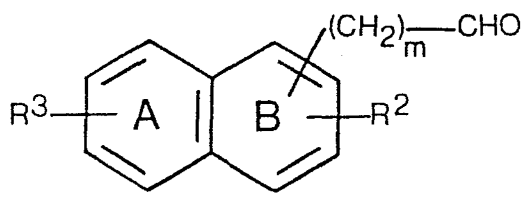

V

worin $R^2$, $R^3$, A, B und m die oben angegebenen Bedeutungen besitzen,

mit einem Grignard-Reagenz der Formel

$R^4$-MgZ VI

worin $R^4$ nieder Alkyl, nieder Alkenyl, nieder Alkinyl oder Phenyl, wobei der Phenylrest gegebenenfalls mit Halogen, Methyl, Aethyl, Methoxy, Aethoxy und/oder Nitro substituiert sein kann, und Z Chlor, Brom oder Jod bedeutet,

unter den Bedingungen einer Grignard-Reaktion umsetzt; oder für den Fall, dass n die Zahl 0 bedeutet, m also 1, 2 oder 3 ist, indem man einen Ester der Formel

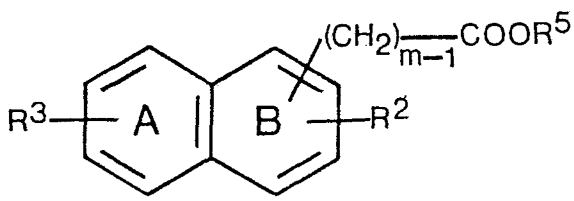

VII

worin $R^2$, $R^3$,m, A und B die oben angegebenen Bedeutungen besitzen und $R^5$ nieder Alkyl bedeutet,

mit einem komplexen Metallhydrid, beispielsweise Lithiumaluminiumhydrid, in einem inerten organischen Lösungsmittel, vorzugsweise Diäthyläther oder Tetrahydrofuran, in einem Temperaturbereich zwischen -10°C und dem Siedepunkt des Reaktionsgemisches reduziert.

Zur Herstellung der Ausgangsmaterialien der Formel IV können die Alkohole der Formel III' in üblicher Weise in die Verbindungen der Formel IV übergeführt werden. Zu diesem Zweck behandelt man den Alkohol der Formel III' mit beispielsweise Thionylchlorid oder Phosphortribromid in einem inerten Lösungsmittel, wie beispielsweise Hexan, Benzol oder Diäthyläther, oder mit Methansulfonsäurechlorid oder p-Toluolsulfonsäurechlorid, ebenfalls in einem inerten Lösungsmittel, wie beispielsweise Diäthyläther, in Gegenwart eines Säurebindemittels, wie beispielsweise Pyridin. Die erhaltene Verbindung der Formel IV wird dann mit der Cyclopropanthiocarbonsäure zu einer Verbindung der Formel I gemäss Verfahrensvariante b) umgesetzt.

Die vorliegende Erfindung betrifft auch pestizide Zusammensetzungen, die als aktiven Bestandteil die Verbindungen der Formel I, wie oben definiert, enthalten. Die pestizide Zusammensetzung enthält zweckmässigerweise zumindest eines der folgenden Materialien: Trägerstoffe, Netzmittel, inerte Verdünnungsmittel und Lösungsmittel.

Die vorliegende Erfindung betrifft ferner die Behandlung von Pflanzen, von Tieren und des Erdbodens sowie von Gegenständen und Flächen, welche darin besteht, dass auf diese die Verbindungen der Formel I, wie oben definiert, aufgebracht werden.

Die Verbindungen der Formel I sind somit ganz allgemein als Pestizide wertvoll. Sie zeigen sich besonders wertvoll als Insektizide und Akarizide, insbesondere gegen saugende Schädlinge wie weisse Fliegen, Thrips und Spinnentiere, beispielsweise Tetranychidae, Eriophyidae und Ixodoidea. Die Verbindungen wirken vorwiegend als direkte Ovizide und reduzieren auch längerfristig den Populationsanstieg. Die Verbindungen haben systemische Aktivität. Die akarizide Wirkung stellt einen bevorzugten Aspekt der vorliegenden Erfindung dar.

Die Verbindungen der Formel I sind im allgemeinen wasserunlöslich und können nach irgendeiner der für unlösliche Verbindungen üblichen Methoden konfektioniert werden.

Wenn gewünscht, können die Verbindungen der Formel I in einem mit Wasser nicht mischbaren Lösungsmittel, wie beispielsweise einem hochsiedenden Kohlenwasserstoff, gelöst werden, das zweckmässigerweise gelöste Emulgatoren enthält, so dass es bei Zugabe zu Wasser als selbstemulgierbares Oel wirkt.

Die Verbindungen der Formel I können auch mit einem Netzmittel mit oder ohne inertes Verdünnungsmittel zur Bildung eines benetzbaren Pulvers vermischt werden, welches in Wasser löslich oder dispergierbar ist, oder sie können mit dem inerten Verdünnungsmittel zur Bildung eines festen oder pulverförmigen Produktes vermischt werden.

Inerte Verdünnungsmittel, mit welchen die Verbindungen der Formel I verarbeitet werden können, sind feste inerte Medien, einschliesslich pulverförmige oder feinverteilte Feststoffe, wie beispielsweise Tone, Sande, Talk, Glimmer, Düngemittel und dgl., wobei solche Produkte entweder staubförmig oder als Materialien mit grösserer Teilchengrösse vorliegen können.

Die Netzmittel können anionische Verbindungen, wie beispielsweise Seifen, Fettsulfatester, wie Dodecylnatriumsulfat, Octadecylnatriumsulfat und Cetylnatriumsulfat, fettaromatische Sulfonate, wie Alkylbenzolsulfonate oder Butylnaphthalinsulfonate, komplexere Fettsulfonate, wie die Amidkondensationsprodukte von Oelsäure und N-Methyltaurin oder das Natriumsalz des Sulfobernsteinsäure-bis-2-äthylhexylesters sein.

Die Netzmittel können auch nichtionische Netzmittel,

wie beispielsweise Kondensationsprodukte von Fettsäuren, Fettalkoholen oder fettsubstituierten Phenolen mit Aethylenoxyd, oder Fettsäureester und -äther von Zuckern oder mehrwertigen Alkoholen oder die Produkte, die aus letzteren durch Kondensation mit Aethylenoxyd erhalten werden, oder die Produkte, die als Blockcopolymere von Aethylenoxyd und Propylenoxyd bekannt sind, sein. Die Netzmittel können auch kationische Mittel, wie beispielsweise Cetyltrimethylammoniumchlorid und dgl., sein.

Die pestizide Zusammensetzung kann auch in Form einer Aerosolverbindung vorliegen, wobei zweckmässigerweise zusätzlich zum Treibgas, welches geeigneterweise ein polyhalogeniertes Alkan, wie Dichlordifluormethan ist, ein Co-Solvens und ein Netzmittel verwendet wird.

Die pestiziden Zusammensetzungen gemäss der vorliegenden Erfindung können zusätzlich zu den Verbindungen der Formel I Synergisten und andere aktive Insektizide, Bakterizide und Fungizide enthalten.

In ihren verschiedenen Anwendungsgebieten können die erfindungsgemässen Verbindungen in unterschiedlichen Mengenverhältnissen eingesetzt werden; so wird beispielsweise für die Behandlung von Pflanzen zur Bekämpfung von Schädlingen auf diesen die Verbindungen zweckmässigerweise in einem Ausmass von etwa 100-2000 g/ha verwendet; für die Behandlung von Tieren zur Bekämpfung von Ectoparasiten wird das Tier zweckmässigerweise in eine Lösung, enthaltend 100-1000 ppm aktiver Verbindung, getaucht oder mit dieser besprüht.

Die Verbindungen besitzen eine akute Toxizität von über 1000 mg/kg. Sie sind somit für Wirbeltiere äusserst wenig toxisch.

Ein weiterer Aspekt der vorliegenden Erfindung ist

die hohe UV-Stabilität der Verbindungen der Formel I.

Die folgenden Beispiele sollen die vorliegende Erfindung illustrieren. Alle Temperaturen sind in °C angegeben.

Beispiel 1

2 g 1-Hydroxymethylnaphthalin werden in 20 ml Benzol und 0,7 ml Pyridin gelöst. Nach Zugabe von 1,2 ml Cyclopropancarbonsäurechlorid in 10 ml Benzol wird die Mischung 15 Minuten auf 70° erhitzt. Das Reaktionsprodukt wird in Wasser gegossen und mit Aether extrahiert. Der Aetherextrakt wird mit Wasser gewaschen, über Natriumsulfat getrocknet und unter vermindertem Druck eingedampft. Der zurückbleibende Cyclopropancarbonsäure-1-naphthylmethylester kann durch Adsorption an Kieselgel gereinigt werden; $n_D^{20}$: 1,5924.

In analoger Weise erhält man bei Einsatz von:

1-Naphthyläthanol und Cyclopropancarbonsäurechlorid den Cyclopropancarbonsäure-2-(1-naphthyl)äthylester; $n_D^{20}$: 1,5855;

2-Naphthyläthanol und Cyclopropancarbonsäurechlorid den Cyclopropancarbonsäure-2-(2-naphthyl)äthylester; $n_D^{20}$: 1,5758;

(1,2,3,4-Tetrahydro-2-naphthyl)methanol und Cyclopropancarbonsäurechlorid den Cyclopropancarbonsäure-(1,2,3,4-tetrahydro-2-naphthyl)methylester; $n_D^{20}$: 1,5370;

1-(2-Naphthyl)äthanol und Cyclopropancarbonsäurechlorid den Cyclopropancarbonsäure-1-(2-naphthyl)äthylester; $n_D^{20}$: 1,5760;

(2-Decahydronaphthyl)methanol und Cyclopropancarbonsäurechlorid den Cyclopropancarbonsäure-(2-decahydronaphthyl)methylester; $n_D^{25}$: 1,5355;

3-(5,6,7,8-Tetrahydro-1-naphthyl)propanol und Cyclopropancarbonsäurechlorid den Cyclopropancarbonsäure-3-

(5,6,7,8-tetrahydro-1-naphthyl)propylester; $n_D^{25}$: 1,5375;

(1-Brom-2-naphthyl)methanol und Cyclopropancarbonsäurechlorid den Cyclopropancarbonsäure-(1-brom-2-naphthyl)methylester; $n_D^{20}$: 1,5940;

(3-Methoxy-2-naphthyl)methanol und Cyclopropancarbonsäurechlorid den Cyclopropancarbonsäure-(3-methoxy-2-naphthyl)methylester; $n_D^{20}$: 1,5890;

(5,6,7,8-Tetrahydro-2-naphthyl)methanol und Cyclopropancarbonsäurechlorid den Cyclopropancarbonsäure-(5,6,7,8-tetrahydro-2-naphthyl)methylester; $n_D^{25}$: 1,5355;

(4-Brom-1-naphthyl)methanol und Cyclopropancarbonsäurechlorid den Cyclopropancarbonsäure-(4-brom-1-naphthyl)methylester; Fp.: 55-56°;

(2-Methoxy-1-naphthyl)methanol und Cyclopropancarbonsäurechlorid den Cyclopropancarbonsäure-(2-methoxy-1-naphthyl)methylester; Fp.: 59-63°;

(1-Methoxy-2-naphthyl)methanol und Cyclopropancarbonsäurechlorid den Cyclopropancarbonsäure-(1-methoxy-2-naphthyl)methylester; $n_D^{20}$: 1,5857;

(6-Methoxy-2-naphthyl)methanol und Cyclopropancarbonsäurechlorid den Cyclopropancarbonsäure-(6-methoxy-2-naphthyl)methylester; Fp.: 81-83°;

α-(Trichlormethyl)-2-naphthalinmethanol und Cyclopropancarbonsäurechlorid den Cyclopropancarbonsäure-2,2,2-trichlor-1-(2-naphthyl)äthylester; Fp.: 108-109°.

## Beispiel 2

3,6 g 1-(2-Naphthyl)-2-propin-1-ol und 1,6 g Pyridin

werden in 30 ml Methylenchlorid gelöst und bei 0-5° unter Rühren mit einer Lösung von 2,3 g Cyclopropancarbonsäurechlorid in 10 ml Methylenchlorid versetzt. Dann lässt man 2 Stunden bei Raumtemperatur reagieren. Das Reaktionsgemisch wird auf Wasser gegossen und mit 50 ml Methylenchlorid verdünnt. Die Methylenchloridlösung wird abgetrennt und der Reihe nach mit 2N Salzsäurelösung, 10%iger Kaliumbicarbonatlösung und halbgesättigter Kochsalzlösung gewaschen, über Natriumsulfat getrocknet und eingedampft. Das Rohprodukt kann durch Kristallisation oder durch Adsorptionschromatographie an Kieselgel gereinigt werden. Man erhält reinen
Cyclopropancarbonsäure-1-(2-naphthyl)-2-propinylester; Fp.: 94-96° (Cyclohexan).

In Analogie zu dem im vorstehenden Beispiel angegebenen Verfahren erhält man aus:

2-Naphthaldehydcyanhydrin und Cyclopropancarbonsäurechlorid den Cyclopropancarbonsäure-α-cyano-2-naphthylmethylester; Fp.: 65-66°,

α-Isopropyl-2-naphthalinmethanol und Cyclopropancarbonsäurechlorid den Cyclopropancarbonsäure-2-methyl-1-(2-naphthyl)propylester; Fp.: 35-37°,

α-Phenyl-2-naphthalinmethanol und Cyclopropancarbonsäurechlorid den Cyclopropancarbonsäure-α-2-naphthylbenzylester; $n_D^{20}$: 1,6004.

Beispiel 3

1,89 g Cyclopropancarbonsäure werden in 50 ml Aceton gelöst, mit 5,5 g Kaliumcarbonat versetzt und auf 50° aufgeheizt. Dann lässt man eine Lösung von 4,7 g 6-Methyl-2-(brommethyl)naphthalin in 20 ml Aceton zutropfen und lässt während 15 Stunden bei 65° reagieren. Das erkaltete Reaktionsgemisch wird filtriert, der Rückstand mit Aceton nachgewaschen und das Filtrat eingedampft. Der Rückstand kann durch Chromatographie an Kieselgel mit Hexan-Essigester (9:1) gereinigt werden. Man erhält reinen Cyclopropancarbonsäure-(6-methyl-2-naphthyl)methylester; Fp.: 75-78° (Pentan-Aether).

In Analogie zu dem im vorstehenden Beispiel angegebenen Verfahren erhält man aus:

3-Methyl-2-(brommethyl)naphthalin und Cyclopropancarbonsäure den Cyclopropancarbonsäure-(3-methyl-2-naphthyl)-methylester; Fp.: 60-61° (Hexan).

## Beispiel 4

3,28 g Magnesium werden mit einem Jod-Kristall versetzt und mit absolutem Diäthyläther überschichtet. Durch Zugabe von 2 g Allylbromid wird die Reaktion in Gang gebracht. Dann tropft man ein Gemisch von 19,78 g Allylbromid und 15,62 g 2-Naphthaldehyd in absolutem Diäthyläther so zu, dass das Reaktionsgemisch am Rückflussieren bleibt. Nach erfolgter Zugabe lässt man über Nacht bei Raumtemperatur rühren. Das Reaktionsgemisch wird auf ein Gemisch aus Eis und 2N Salzsäurelösung gegossen und dreimal mit Aether extrahiert. Die Aetherextrakte werden mit Wasser und gesättigter Kochsalzlösung gewaschen, über Natriumsulfat getrocknet und eingedampft. Man erhält 1-(2-Naphthyl)-3-buten-1-ol, das ohne weitere Reinigung zur Reaktion mit Cyclopropancarbonsäurechlorid, gemäss dem in Beispiel 2 beschriebenen Verfahren, eingesetzt werden kann. Man erhält den Cyclopropancarbonsäure-1-(2-naphthyl)-3-butenylester; Fp.: 35-39°.

## Beispiel 5

1,8 g Magnesium werden mit 10 ml absolutem Tetrahydrofuran überdeckt. Dann lässt man ca. 1 ml einer Lösung von 8 g Vinylbromid in 8 ml absolutem Tetrahydrofuran zutropfen und bringt die Reaktion mittels leichtem Erwärmen in Gang. Anschliessend lässt man den Rest der Lösung zutropfen und lässt solange reagieren bis das Reaktionsgemisch Raumtemperatur angenommen hat. Danach lässt man eine Lösung von 7 g 2-Naphthaldehyd in 50 ml abs. Tetrahydrofuran langsam zutropfen, wobei die Reaktionstemperatur auf 40° ansteigt. Innert einer Stunde erhält man eine gute Umsetzung. Das Reaktionsgemisch wird bei 0° unter gutem Rühren durch Zugabe von 20 ml gesättigter Ammoniumchloridlösung hydrolysiert. Der Rückstand wird abfiltriert und mit Aether ausgewaschen. Das Filtrat wird mit Wasser verdünnt und zweimal mit Aether extrahiert. Die Extrakte werden mit halbgesättigter und gesättigter Kochsalzlösung gewaschen, über Natriumsulfat getrocknet und eingedampft. Man erhält 1-(2-Naphthyl)-2-propen-1-ol, das ohne weitere Reinigung, gemäss dem in Beispiel 2 angegebenen Verfahren, mit Cyclopropancarbonsäurechlorid verestert wird. Man erhält den Cyclopropancarbonsäure-1-(2-naphthyl)allylester; Fp.: 30-32°.

## Beispiel 6

6,5 g 6-Methoxy-2-naphthaldehyd werden mit einer Lösung von 4,28 g Natriumcyanid in 115 ml 95%igem Aethanol versetzt. Dann tropft man während 1 Stunde 11,5 ml Essigsäure bei 0° zu und lässt über Nacht reagieren. Zur Aufarbeitung werden 400 ml Wasser zugegeben und danach wird dreimal mit Aether extrahiert. Die Extrakte werden mit 10%iger Kaliumbicarbonatlösung, halbgesättigter und gesättigter Kochsalzlösung gewaschen, über Natriumsulfat getrocknet und eingedampft. Man erhält α-Cyano(6-methoxy-2-naphthyl)methanol, der ohne weitere Reinigung, gemäss dem in Beispiel 2 angegebenen Verfahren, mit Cyclopropancarbonsäurechlorid umgesetzt wird. Man erhält den Cyclopropancarbonsäure-α-cyano-(6-methoxy-2-naphthyl)methyl-

ester; $n_D^{20}$: 1,5765.

Beispiel 7

In einer Suspension von 2,09 g Lithiumaluminiumhydrid in 200 ml absolutem Tetrahydrofuran werden bei -10° unter Stickstoff-Begasung 14,55 g 1-(2-Propinyloxy)-naphthoesäure-2-propinylester gelöst in 50 ml abs. Tetrahydrofuran vorsichtig zugegeben. Nach erfolgter Zugabe lässt man noch 1 Stunde bei ca. 0° reagieren. Zur Hydrolyse wird unter Rühren bei -10° vorsichtig tropfenweise Wasser zugegeben, wobei man die Temperatur nicht über 0° ansteigen lässt. Das Reaktionsgemisch wird über ein Filterhilfsmittel (Cellit) filtriert und das Filtrat eingeengt. Der Rückstand wird mit Essigester aufgenommen und der Reihe nach mit 2N Salzsäurelösung, 10%iger Kaliumbicarbonatlösung, halbgesättigter und gesättigter Kochsalzlösung gewaschen, über Natriumsulfat getrocknet und eingedampft. Durch Umkristallisieren erhält man reinen [1-(2-Propinyloxy)-2-naphthyl]methanol; Fp.: 65-68° (Hexan-Essigester), der analog zu dem in Beispiel 2 angegebenen Verfahren mit Cyclopropancarbonsäurechlorid umgesetzt wird. Man erhält den Cyclopropancarbonsäure-[1-(2-propinyloxy)-2-naphthyl]methylester; $n_D^{20}$: 1,5902.

Beispiel 8

20,2 g 3-Hydroxy-2-naphthoesäure-methylester werden in 200 ml Methyläthylketon gelöst und unter Rühren und $N_2$-Begasung mit 27,6 g Kaliumcarbonat und 23,6 g Propargylbromid versetzt. Das heterogene Reaktionsgemisch wird über Nacht am Rückfluss gekocht und dann filtriert. Das Filtrat wird im Vakuum eingeengt, der Rückstand auf 250 ml Eiswaser gegossen und dreimal mit Hexan-Essigester (1:1) extrahiert. Die Extrakte werden mit 2N Salzsäurelösung, 10%iger Kaliumbicarbonatlösung, halbgesättigter

und gesättigter Kochsalzlösung gewaschen, getrocknet und am Rotationsverdampfer eingedampft. Man erhält den 3-(2-Propinyloxy)-2-naphthoesäure-methylester, der ohne weitere Reinigung, gemäss dem in Beispiel 7 angegebenen Verfahren, mittels Lithiumaluminiumhydrid reduziert wird. Man erhält [3-(2-Propinyloxy)-2-naphthyl]methanol, der seinerseits direkt, gemäss dem in Beispiel 2 angegebenen Verfahren, mit Cyclopropancarbonsäurechlorid umgesetzt wird. Man erhält den Cyclopropancarbonsäure-[3-(2-propinyloxy)-2-naphthyl]methylester; Fp.: 58-60°.

Beispiel 9

2-(2-Propinyloxy)-1-naphthaldehyd und Lithiumaluminiumhydrid werden gemäss dem in Beispiel 7 beschriebenen Verfahren umgesetzt. Man erhält [2-(2-Propinyloxy)-1-naphthyl]methanol; Fp.: 37-43°, der analog zu dem im Beispiel 2 beschriebenen Verfahren mit Cyclopropancarbonsäurechlorid umgesetzt wird. Man erhält den Cyclopropancarbonsäure-[2-(2-propinyloxy)-1-naphthyl]methylester; Fp.: 69-73°.

Beispiel 10

4,0 g 2-Naphthylmethylthiol und 2,4 g Cyclopropancarbonsäurechlorid werden in 50 ml Toluol vorgelegt. Nach tropfenweiser Zugabe von 2,4 g Triäthylamin in 20 ml Toluol wird das Gemisch 20 Stunden auf 85° erhitzt. Das Reaktionsgemisch wird in Wasser gegossen und mit Aether extrahiert. Der Aetherextrakt wird mit Wasser gewaschen, über Natriumsulfat getrocknet, filtriert und eingedampft. Durch Chromatographie an Kieselgel mit Hexan-Aether (19:1) wird reiner Cyclopropanthiolcarbonsäure-2-naphthylmethylester erhalten. Fp.: 39-40°.

In Analogie zu dem im vorstehenden Beispiel angegebenen Verfahren erhält man aus 1-Naphthylmethylthiol und

Cyclopropancarbonsäurechlorid den Cyclopropanthiolcarbonsäure-1-naphthylmethylester; $n_D^{25}$: 1,3359.

Beispiel 11

2,6 g Cyclopropacarbonsäurechlorid werden während 5 Minuten zu einer eisgekühlten Lösung von 2,0 g Natriumhydrogensulfid in 20 ml Aethanol gegeben. Nach 30 Minuten werden 5,5 g 2-Brommethylnaphthalin zugegeben, worauf bei Raumtemperatur langsam 10 ml 10%ige Natronlauge zugetropft werden. Nach 1-stündigem Rühren bei Raumtemperatur werden 25 ml Dichlormethan zugegeben und zweimal mit 10%iger Kaliumbicarbonatlösung und gesättigter Kochsalzlösung gewaschen, über Natriumsulfat getrocknet und eingedampft. Durch Chromatographie an Kieselgel wird reiner Cyclopropanthiolcarbonsäure-2-naphthylmethylester erhalten.

Beispiel 12

In Analogie zu dem in Beispiel 3 angegebenen Verfahren erhält man aus Cyclopropancarbonsäure und 1-Nitro-2-(brommethyl)-naphthalin den Cyclopropancarbonsäure-(1-nitro-2-naphthyl)methylester; Fp.: 84-86°.

Zur Herstellung des Ausgangsmaterials wird ein Gemisch aus 18,7 g 2-Methyl-1-nitro-naphthalin, 17,8 g N-Bromsuccinimid, 400 ml Tetrachlorkohlenstoff und 0,1 g Dibenzoylperoxyd mittels einer Photolampe zum Sieden erhitzt. Nach erfolgter Reaktion wird das abgekühlte Reaktionsgemisch abgenutscht. Das Filtrat wird auf Wasser gegossen und mit Methylenchlorid extrahiert. Die Extrakte werden der Reihe nach mit 10%iger Natronlauge, Wasser und Kochsalzlösung gewaschen, über Natriumsulfat getrocknet und eingedampft. Man erhält rohes 1-Nitro-2-(brommethyl)-naphthalin, das zur Reaktion eingesetzt werden kann.

Beispiel 13

Die Verbindungen der Formel I können für die biologische Prüfung beispielsweise wie folgt formuliert werden:

| Emulsionskonzentrat | | g/l |
|---|---|---|
| Wirkstoff der Formel I | | 500 |
| Mischung von Rizinusöl/Aethylenoxid-Kondensationsprodukt mit ca. 25 Mol Aethylenoxid und Ca-Dodecylbenzolsulfonat im Verhältnis 3:1 | | 100 |
| Epoxidiertes Soyaöl mit einem Oxiran-Sauerstoffgehalt von 6% | | 25 |
| Butyliertes Hydroxytoluol | | 10 |
| Lösungsmittel bestehend aus einer Mischung aus Mono-, Di- und Tri-nieder Alkylbenzolen | ad | 1000 ml |

| Spritzpulver | |
|---|---|
| Wirkstoff der Formel I | 50 |
| Hydratisierte Kieselsäure | 37 |
| Maleinsäure-Diisobutylen-Copolymer-Na-Salz | 4 |
| Nonylphenol-äthoxylat | 4 |
| Kaolin | 5 |
| | 100,0 g |

In den nachstehenden biologischen Beispielen entsprechen die römischen Zahlen den folgenden Verbindungen:

| | |
|---|---|
| I | Cyclopropancarbonsäure-1-naphthylmethylester |
| II | Cyclopropancarbonsäure-2-(1-naphthyl)äthylester |

III Cyclopropancarbonsäure-2-(2-naphthyl)äthylester

IV Cyclopropancarbonsäure-(5,6,7,8-tetrahydro-2-naphthyl)methylester

V Cyclopropancarbonsäure-(1,2,3,4-tetrahydro-2-naphthyl)methylester

VI Cyclopropancarbonsäure-1-(2-naphthyl)äthylester

VII Cyclopropancarbonsäure-(decahydro-2-naphthyl)-methylester

VIII Cyclopropancarbonsäure-(2-methoxy-1-naphthyl)-methylester

IX Cyclopropancarbonsäure-[2-(2-propinyloxy)-1-naphthyl]methylester

X Cyclopropancarbonsäure-(1-methoxy-2-naphthyl)-methylester

XI Cyclopropancarbonsäure-3-(5,6,7,8-tetrahydro-1-naphthyl)propylester

XII Cyclopropancarbonsäure-α-cyano-2-naphthyl-methylester

XIII Cyclopropancarbonsäure-1-(2-naphthyl)-2-propinylester

XIV Cyclopropancarbonsäure-(6-methoxy-2-naphthyl)-methylester

XV Cyclopropancarbonsäure-2,2,2-trichlor-1-(2-naphthyl)äthylester

XVI Cyclopropancarbonsäure-1-(2-naphthyl)-3-butenylester

XVII Cyclopropancarbonsäure-(4-brom-1-naphthyl)-methylester

XVIII Cyclopropancarbonsäure-(3-methoxy-2-naphthyl)-methylester

XIX Cyclopropancarbonsäure-α-2-naphthylbenzylester

XX Cyclopropancarbonsäure-(6-methyl-2-naphthyl)-methylester.

Beispiel 14

Testorganismus: Tetranychus urticae, gemeine Spinnmilbe.

Testmethode: Buschbohnen-Blattrondellen, infiziert mit 20 mobilen Spinnmilben, werden mit acetonischer Wirkstofflösung besprüht. Die behandelten Blattrondellen werden auf feuchten Schaumstoff gelegt und bei 25$^{o}$ und 60% relativer Feuchtigkeit inkubiert. Unbehandelte und mit Aceton behandelte Rondellen dienen als Kontrollen. Testdauer: 3 Tage. Die Resultate werden ausgedrückt als prozentuale Reduktion der Ueberlebensrate im Vergleich zu den Kontrollparzellen.

| Dosierung ($10^{-x}$g AS/$cm^2$): | 5 | 6 |
|---|---|---|
| | % Wirkung | |
| Verbindung I | 100 | 0 |
| II | 100 | 0 |
| III | 100 | 0 |
| IV | 100 | 7 |
| V | 100 | 21 |
| VI | 97 | 2 |
| VII | 100 | 6 |
| VIII | 100 | 0 |
| IX | 100 | 3 |
| X | 100 | 14 |
| XI | 100 | 8 |
| XII | 100 | 18 |
| XIII | 100 | 40 |
| XIV | 100 | 48 |
| XV | 100 | 20 |
| XVI | 100 | 1 |
| XVII | 100 | 0 |

(Mortalität in den unbehandelten Kontrollparzellen: 5%)

AS = Aktivsubstanz

## Beispiel 15

Testorganismus: Tetranychus urticae, gemeine Spinnmilbe.

Testmethode: Junge Buschbohnenpflanzen (2-Blatt-Stadium) werden mit acetonischer Wirkstofflösung besprüht. Nach der Behandlung werden die Pflanzen während 7 Tagen bei 28° und 60% relativer Feuchtigkeit unter UV-Licht (Philips TLA 40W/05) inkubiert. Nach dieser Zeit werden auf, aus den Blättern ausgestanzte, Rondellen (Ø 25 mm) 8-10 adulte Weibchen angesetzt. 1 Tag später werden die Weibchen entfernt und die Rondellen mit 30-50 Eiern werden bis zum Schlüpfen der Larven bei 25° und 60% relativer Feuchtigkeit inkubiert. Unbehandelte und mit Aceton behandelte Pflanzen (resp. Rondellen daraus) dienen als Kontrollen. Testdauer: 7 Tage Vorinkubation unter UV + 6 Tage. Die Resultate werden ausgedrückt als prozentuale Reduktion der Ueberlebensrate im Vergleich zu den Kontrollen.

| Dosierung ($10^{-x}$g AS/$cm^2$): | 5 | 6 | 7 |
|---|---|---|---|
| | | % Wirkung | |
| Verbindung VI | 100 | 0 | |
| XI | 94 | 0 | |
| XII | 100 | 0 | |
| XIII | 100 | 0 | |
| XIV | 100 | 94 | 13 |
| XVI | 100 | 32 | 0 |
| XVII | 88 | 0 | |
| XVIII | 100 | 0 | |
| XIX | 100 | 100 | 0 |
| XX | 100 | 100 | 8 |

(Mortalität in den unbehandelten Kontrollparzellen: 3%)

AS = Aktivsubstanz

Beispiel 16

Die Verbindungen der Formel I können wie folgt formuliert werden:

| | g/Liter |
|---|---|
| Wirkstoff der Formel I | 500 |
| N-Methyl-2-pyrrolidon | 300 |
| Emulgator-Mischung aus Calcium-Alkylarylsulfonat, Alkylphenoläthoxylat, Blockpolymerisat aus Propylenoxid und Aethylenoxid | 100 |
| Calcium-Dodecylbenzolsulfonat | 25 |
| Lösungsmittel bestehend aus einer Mischung aus Mono-, Di- und Tri-nieder Alkylbenzolen | ad 1000 ml |

Erläuterung der in den nachstehenden Beispielen verwendeten Abkürzungen:

| Verbindung: (Abkürzung) | Strukturformel: |
|---|---|
| Formel I | 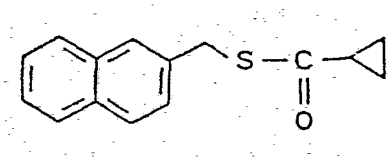 Cyclopropanthiolcarbonsäure-2-naphthylmethylester |

Standards

| | |
|---|---|
| Verbindung A (ZARDEX) | 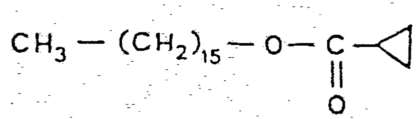 Cyclopropancarbonsäure-hexadecylester |

Verbindung B
(Thionazin, NEMAFOS)

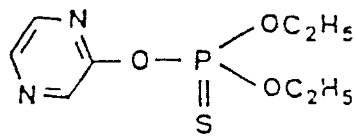

O-(2-Pyrazinyl)-thiophosphorsäure-O,O-diäthylester

Beispiel 17

Testorganisums: Tetranychus urticae, gemeine Spinnmilbe.

Testmethode: wie im Beispiel 15.

| Dosis ($10^{-x}$ g AS/$cm^2$): | 5 | 6 | 7 |
|---|---|---|---|
| | | % Wirkung | |
| (Unbehandelte Kontrollen: 3% Mortalität) | | | |
| Formel I | 98 | 78 | 22 |
| Verbindung A | 100 | 0 | 0 |

AS = Wirkstoff

Beispiel 18

Testorganismus: Trialeurodes vaporariorum, Weisse Fliege.

Testmethode: Buschbohnen-Blattrondellen (Ø 25 mm) mit Eiern (0-15 Stunden alt) werden mit acetonischer Wirkstofflösung besprüht. Die Blattrondellen werden auf feuchtes Filterpapier gelegt und bei 25° und 60% rel. Feuchtigkeit bis zum Schlüpfen der Larven inkubiert. Unbehandelte und mit Aceton behandelte Rondellen dienen als Kontrollen. Testdauer: 9 Tage. Die Resultate werden ausgedrückt als prozentuale Reduktion der Ueberlebensrate im Vergleich zu den Kontrollen.

| Dosis ($10^{-x}$ g AS/$cm^2$): | 5 | 6 | 7 | 8 |
|---|---|---|---|---|
| | | % Wirkung | | |
| (Unbehandelte Kontrollen: 12% Mortalität) | | | | |
| Formel I | 100 | 100 | 52 | 0 |
| Verbindung B | 23 | 0 | 0 | |

AS = Wirkstoff

## Patentansprüche

1. Verbindungen der Formel

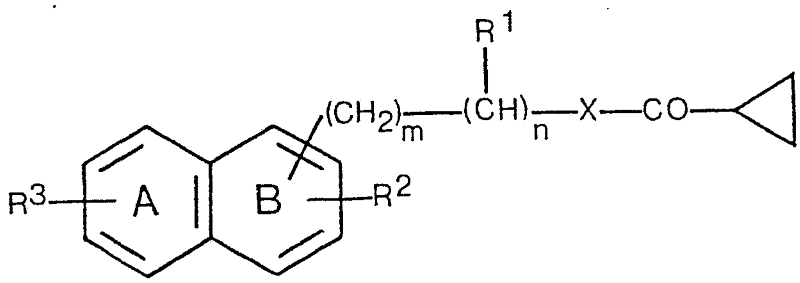

I

worin $R^1$ nieder Alkyl, mit Halogen substituiertes nieder Alkyl, nieder Alkenyl, nieder Alkinyl, Cyan oder Phenyl, wobei der Phenylrest gegebenenfalls mit Halogen, Methyl, Aethyl, Methoxy, Aethoxy und/oder Nitro substituiert sein kann; $R^2$ Wasserstoff, Halogen, Alkyl mit 1 bis 3 Kohlenstoffatomen, Alkoxy mit 1 bis 3 Kohlenstoffatomen, Alkenyloxy mit 3 oder 4 Kohlenstoffatomen, Alkinyloxy mit 3 oder 4 Kohlenstoffatomen oder Nitro; $R^3$ Wasserstoff, Alkyl mit 1 bis 3 Kohlenstoffatomen oder Alkoxy mit 1 bis 3 Kohlenstoffatomen; X Sauerstoff oder Schwefel; m die Zahl 0, 1, 2 oder 3; und n die Zahl 0 oder 1; bedeuten und einer der beiden Ringe A und B oder beide auch gesättigt sein können; wobei jedoch die beiden Symbole m und n aber nicht gleichzeitig die Zahl 0 darstellen; mit der Massgabe, dass die beiden Symbole $R^2$ und $R^3$ nicht gleichzeitig Wasserstoff sind, wenn X Sauerstoff und m die Zahl 1 darstellen und die den Cyclopropan(thiol)carbonsäurerest tragende Methylengruppe mit der ß-Stellung des Naphthalinrestes verknüpft ist.

2. Verbindungen der Formel I in Anspruch 1, worin $R^1$, $R^2$, $R^3$, X, A, B, m und n die in Anspruch 1 angebenen Bedeutungen besitzen, mit der Massgabe, dass die beiden Symbole $R^2$ und $r^3$ nicht gleichzeitig Wasserstoff sind,

wenn m die Zahl 1 darstellt und die den Cyclopropan(thiol)carbonsäurerest tragende Methylengruppe mit der ß-Stellung des Naphthalinrestes verknüpft ist.

3. Verbindungen nach Anspruch 1 oder 2, worin X Sauerstoff bedeutet.

4. Verbindungen nach einem der Ansprüche 1 bis 3, worin $R^2$ Wasserstoff oder Halogen bedeutet.

5. Verbindungen nach einem der Ansprüche 1 bis 4, worin $R^3$ Wasserstoff oder eine Methylgruppe in der 6-Stellung des Naphthalinrestes bedeutet.

6. Cyclopropancarbonsäure-α-2-naphthylbenzylester.

7. Cyclopropancarbonsäure-(6-methyl-2-naphthyl)methylester.

8. Cyclopropanthiolcarbonsäure-2-naphthylmethylester.

9. Eine Verbindung nach Anspruch 1, ausgewählt unter:
Cyclopropancarbonsäure-1-naphthylmethylester,
Cyclopropancarbonsäure-2-(1-naphthyl)äthylester,
Cyclopropancarbonsäure-2-(2-naphthyl)äthylester,
Cyclopropancarbonsäure-(1,2,3,4-tetrahydro-2-naphthyl)methylester,
Cyclopropancarbonsäure-1-(2-naphthyl)äthylester,
Cyclopropancarbonsäure-(2-decahydronaphthyl)methylester,
Cyclopropancarbonsäure-3-(5,6,7,8-tetrahydro-1-naphthyl)propylester,
Cyclopropancarbonsäure-(1-brom-2-naphthyl)methylester,
Cyclopropancarbonsäure-(3-methoxy-2-naphthyl)methylester,
Cyclopropancarbonsäure-(5,6,7,8-tetrahydro-2-naphthyl)methylester,

Cyclopropancarbonsäure-(4-brom-1-naphthyl)methylester, Cyclopropancarbonsäure-(2-methoxy-1-naphthyl)methylester, Cyclopropancarbonsäure-(1-methoxy-2-naphthyl)methylester, Cyclopropancarbonsäure-(6-methoxy-2-naphthyl)methylester, Cyclopropancarbonsäure-2,2,2-trichlor-1-(2-naphthyl)-äthylester, Cyclopropancarbonsäure-1-(2-naphthyl)-2-propinylester, Cyclopropancarbonsäure-α-cyano-2-naphthylmethylester, Cyclopropancarbonsäure-2-methyl-1-(2-naphthyl)propylester, Cyclopropancarbonsäure-(3-methyl-2-naphthyl)methylester, Cyclopropancarbonsäure-1-(2-naphthyl)-3-butenylester, Cyclopropancarbonsäure-1-(2-naphthyl)allylester, Cyclopropancarbonsäure-α-cyano-(6-methoxy-2-naphthyl)-methylester, Cyclopropancarbonsäure-[1-(2-propinyloxy)-2-naphthyl]-methylester, Cyclopropancarbonsäure-[3-(2-propinyloxy)-2-naphthyl]-methylester, Cyclopropancarbonsäure-[2-(2-propinyloxy)-1-naphthyl]-methylester.

10. Eine Verbindung nach Anspruch 1, ausgewählt unter: Cyclopropanthiolcarbonsäure-1-naphthylmethylester und Cyclopropancarbonsäure-(1-nitro-2-naphthyl)methylester.

11. Verbindungen nach einem der Ansprüche 1 bis 10 als Schädlingsbekämpfungsmittel.

12. Schädlingsbekämpfungsmittel, dadurch gekennzeichnet, dass es eine wirksame Menge von mindestens einer Verbindung der allgemeinen Formel

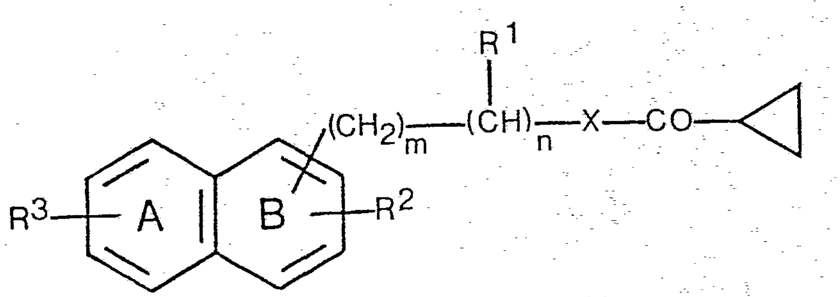

I

worin $R^1$ nieder Alkyl, mit Halogen substituiertes nieder Alkyl, nieder Alkenyl, nieder Alkinyl, Cyan oder Phenyl, wobei der Phenylrest gegebenenfalls mit Halogen, Methyl, Aethyl, Methoxy, Aethoxy und/oder Nitro substituiert sein kann; $R^2$ Wasserstoff, Halogen, Alkyl mit 1 bis 3 Kohlenstoffatomen, Alkoxy mit 1 bis 3 Kohlenstoffatomen, Alkenyloxy mit 3 oder 4 Kohlenstoffatomen, Alkinyloxy mit 3 oder 4 Kohlenstoffatomen oder Nitro; $R^3$ Wasserstoff, Alkyl mit 1 bis 3 Kohlenstoffatomen oder Alkoxy mit 1 bis 3 Kohlenstoffatomen; X Sauerstoff oder Schwefel; m die Zahl 0, 1, 2 oder 3; und n die Zahl 0 oder 1; bedeuten und einer der beiden Ringe A und B oder beide auch gesättigt sein können; wobei jedoch die beiden Symbole m und n aber nicht gleichzeitig die Zahl 0 darstellen; mit der Massgabe, dass die beiden Symbole $R^2$ und $R^3$ nicht gleichzeitig Wasserstoff sind, wenn X Sauerstoff und m die Zahl 1 darstellen und die den Cyclopropan(thiol)carbonsäurerest tragende Methylengruppe mit der ß-Stellung des Naphthalinrestes verknüpft ist,

und inertes Trägermaterial enthält.

13. Schädlingsbekämpfungsmittel nach Anspruch 12, dadurch gekennzeichnet, dass es eine wirksame Menge des Cyclopropancarbonsäure-α-2-naphthylbenzylesters enthält.

14. Schädlingsbekämpfungsmittel nach Anspruch 12, dadurch gekennzeichnet, dass es eine wirksame Menge des Cyclopropancarbonsäure-(6-methyl-2-naphthyl)methylesters enthält.

15. Schädlingsbekämpfungsmittel nach Anspruch 12, dadurch gekennzeichnet, dass es eine wirksame Menge des Cyclopropanthiolcarbonsäure-2-naphthylmethylesters enthält.

16. Verfahren zur Herstellung von Verbindungen der Formel

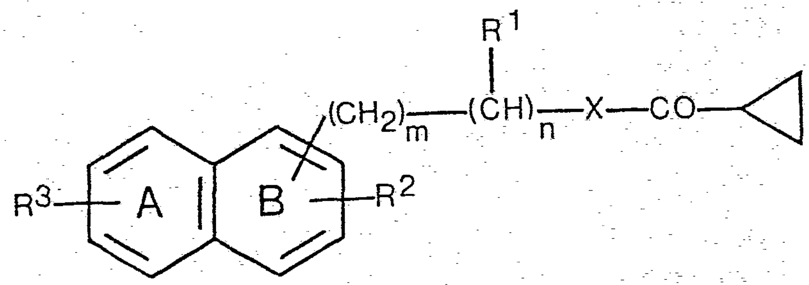

I

worin $R^1$ nieder Alkyl, mit Halogen substituiertes nieder Alkyl, nieder Alkenyl, nieder Alkinyl, Cyan oder Phenyl, wobei der Phenylrest gegebenenfalls mit Halogen, Methyl, Aethyl, Methoxy, Aethoxy und/oder Nitro substituiert sein kann; $R^2$ Wasserstoff, Halogen, Alkyl mit 1 bis 3 Kohlenstoffatomen, Alkoxy mit 1 bis 3 Kohlenstoffatomen, Alkenyloxy mit 3 oder 4 Kohlenstoffatomen, Alkinyloxy mit 3 bis 4 Kohlenstoffatomen oder Nitro; $R^3$ Wasserstoff, Alkyl mit 1 bis 3 Kohlenstoffatomen oder Alkoxy mit 1 bis 3 Kohlenstoffatomen; X Sauerstoff oder Schwefel; m die Zahl 0, 1, 2 oder 3; und n die Zahl 0 oder 1; bedeuten und einer der beiden Ringe A und B oder beide auch gesättigt sein können; wobei jedoch die beiden Symbole m und n aber nicht gleichzeitig die Zahl 0 darstellen; mit der Massgabe, dass die beiden Symbole $R^2$ und $R^3$ nicht Wasserstoff sind, wenn X Sauerstoff und m die Zahl 1 darstellen und die den Cyclopropan(thiol)carbonsäurerest tragende Methylengruppe mit der ß-Stellung des Naphthalinrestes verknüpft ist,

dadurch gekennzeichnet, dass man

a) die Säure oder ein reaktionsfähiges Derivat dieser Säure der Formel

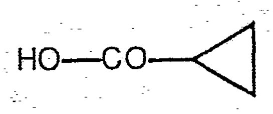

II

mit einem Alkohol bzw. Thioalkohol der Formel

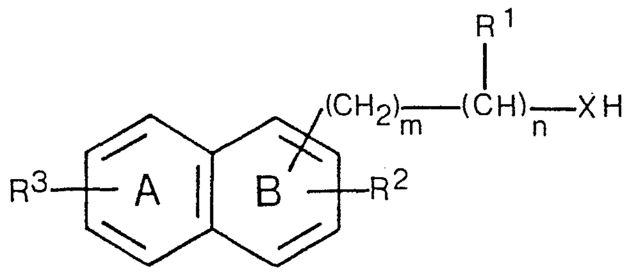

III

worin $R^1$, $R^2$, $R^3$, X, A, B, m und n die oben angegebenen Bedeutungen besitzen,

umsetzt, oder

b) die Säure der Formel II oder die entsprechende Thiolsäure mit einer Verbindung der Formel

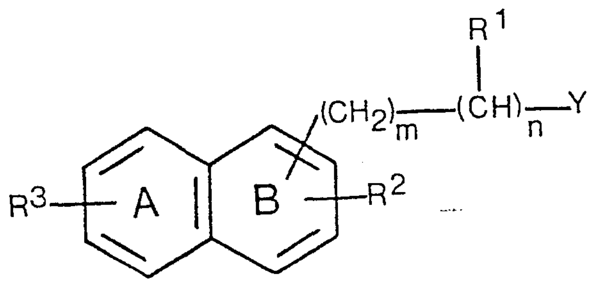

IV

worin $R^1$, $R^2$, $R^3$, A, B, m und n die oben angegebenen Bedeutungen besitzen und Y Chlor, Brom, Mesyloxy oder Tosyloxy bedeutet,

umsetzt.

17. Verfahren zur Bekämpfung von Schädlingen, dadurch gekennzeichnet, dass man die zu schützenden Gegenstände mit einem in den Ansprüchen 12 bis 15 genannten Mittel behandelt.

18. Verwendung einer der in den Ansprüchen 1 bis 10 genannten Verbindungen bzw. eines der in den Ansprüchen 12 bis 15 genannten Mittel zur Bekämpfung von Schädlingen.

****